# EUROPÄISCHE PATENTSCHRIFT

(11) **EP 2 337 549 B1**
(45) Veröffentlichungstag und Bekanntmachung des Hinweises auf die Patenterteilung: **25.12.2013**
(21) Anmeldenummer: 09736203.2
(22) Anmeldetag: 12.10.2009
(51) Int. Cl.: A61K 8/34, A61K 8/33, A61K 8/35, A61K 8/49, A61Q 5/10

(54) **MITTEL ZUM FÄRBEN VON KERATINHALTIGEN FASERN**
AGENTS FOR COLOURING FIBRES CONTAINING KERATIN
PRODUITS POUR COLORER LES FIBRES KÉRATINIQUES

(30) Priorität: 15.12.2008 DE 102008061855; 22.10.2008 DE 102008052676
(43) Veröffentlichungstag der Anmeldung: 29.06.2011
(73) Patentinhaber: Henkel AG & Co. KGaA, 40589 Düsseldorf (DE)
(72) Erfinder: OBERKOBUSCH, Doris, 40591 Düsseldorf (DE); GROß, Wibke, 41836 Hückelhoven (DE); STEPHAN, Laura, 40589 Düsseldorf (DE)
(86) Internationale Anmeldenummer: PCT/EP2009/063240
(87) Internationale Veröffentlichungsnummer: WO 2010/046255

(56) Entgegenhaltungen:
- EP-A2- 2 018 844
- WO-A1-2004/022016
- DE-A1-102004 044 231
- DE-A1-102005 022 790
- DE-A1-102005 026 545
- DE-A1-102005 062 357
- DE-A1-102005 062 834
- DE-A1-102006 060 150

## Beschreibung

Die Erfindung betrifft ein Mittel zum Färben von keratinhaltigen Fasern, insbesondere menschlichen Haaren, das spezielle Kombinationen aus C,H-aciden Verbindungen und reaktiven Carbonylverbindungen enthält, die Verwendung dieser Kombination in Mitteln zum Färben von keratinhaltigen Fasern, zur Farbauffrischung bzw. Nuancierung von bereits gefärbten keratinhaltigen Fasern sowie ein Verfahren zum Färben von keratinhaltigen Fasern, insbesondere menschlichen Haaren. Für das Färben von keratinhaltigen Fasern kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraamino-pyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazol-5-on, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diamino-phenoxyethanol, 2-Amino-4-(2-hydroxyethylamino)-anisol (Lehmanns Blau), 1-Phenyl-3-methyl-pyrazol-5-on, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Mit Oxidationsfarbstoffen lassen sich zwar intensive Färbungen mit guten Echtheitseigenschaften erzielen, die Entwicklung der Farbe geschieht jedoch im allgemeinen unter dem Einfluss von Oxidationsmitteln wie z. B. H₂O₂, was in einigen Fällen Schädigungen der Faser zur Folge haben kann. Problematisch gestaltet sich nach wie vor eine Bereitstellung von Oxidationshaarfärbungen im Rotbereich mit ausreichenden Echtheitseigenschaften, insbesondere in mit sehr guten Wasch- und Reibechtheiten. Des Weiteren können einige Oxidationsfarbstoffvorprodukte beziehungsweise bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, dass die Färbungen häufig nur über unzureichende Echtheitseigenschaften verfügen. Neben den klassischen Haarfarben auf Basis von Oxidationsfarbstoffvorprodukten wurden in den letzten Jahren häufiger Färbesysteme auf Basis von C,H-aciden Verbindungen und reaktiven Carbonylverbindungen beschrieben, die es erlauben, ohne den Einsatz von Wasserstoffperoxid und ohne Verwendung von aromatischen Aminen eine breite Palette von Haarfarben erzielen. WO 2004/022016 beschreibt Mittel zum Färben von Kerakinhaltigen Fasern, die 1,2-Dihydropyridinium-Derivate in Kombination mit reaktiven Carbonyl verbindungen enthalten. Speziell im Gelbbereich bestand jedoch noch Optimierungsbedarf, da die bisher erzielbaren Gelbtöne nicht intensiv genug waren. Gelbtöne von ausreichender Intensität sind erforderlich, um Nuancen im Rot-, Kupfer-, Braun- und Schwarzbereich zu formulieren.

Neben dem mangelnden Färbevermögen der Farbstoffe an sich kann ein unzureichender Farbaufzug auch durch schlechte Löslichkeit der zur Erzielung der gelben Farbe erforderlichen Farbstoffvorprodukte in Gegenwart der übrigen Rezepturbestandteile zustande kommen. Ferner kann die Reaktivität der Aldehyde für den Gelbbereich durch andere Aldehyde in der Färbemischung herabgesetzt werden. Überraschenderweise wurde nun gefunden, dass sich spezielle Kombinationen aus C,H-aciden Verbindungen und reaktiven Carbonylen auch in Abwesenheit von oxidierenden Agentien hervorragend zum Färben von keratinhaltigen Fasern eignen. Sie ergeben Ausfärbungen mit hervorragender Brillanz und Farbtiefe und führen zu vielfältigen Farbnuancen. Es werden auch ohne den Einsatz von Oxidationsmitteln insbesondere Ausfärbungen mit verbesserten Wasch- und Lichtechtheitseigenschaften insbesondere im Gelbbereich erhalten. Der Einsatz von oxidierenden Agentien soll jedoch nicht prinzipiell ausgeschlossen werden. Durch Kombination der erfindungswesendlichen Komponenten mit weiteren C,H-aciden Verbindungen und reaktiven Carbonylen ist es nunmehr möglich, einen Nuancenbereich von gelb über gelbbraun, orange, braunorange, braun, rot, rotviolett bis hin zu blauviolett, dunkelblau und schwarz abzudecken.

Ein erster Gegenstand der vorliegenden Anmeldung sind daher Mittel zur Färbung keratinischer Fasern, enthaltend in einem kosmetisch akzeptablen Träger mindestens eine C,H-acide Verbindung, ausgewählt aus
- den physiologisch verträglichen Salzen des 1-Allyl-1,2-dihydro-3,4,6-trimethyl-2-oxo-pyrimidiniums, insbesondere 1-Allyl-1,2-dihydro-3,4,6-trimethyl-2-oxo-pyrimidinium-bromid 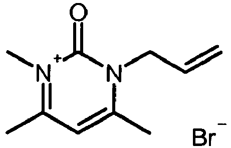 und
- den physiologisch verträglichen Salzen des 1,2-Dihydro-1,3,4,6-tetramethyl-2-oxo-pyrimidimiums, insbesondere 1,2-Dihydro-1,3,4,6-tetramethyl-2-oxo-pyrimidimium-hydrogensulfat, 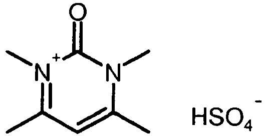 sowie als reaktive Carbonylverbindung mindestens
- 2,3-Dihydro-1,5-dimethyl-3-oxo-2-phenyl-1H-pyrazol-4-carboxaldehyd

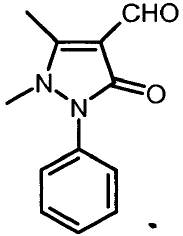

Ebenfalls bevorzugt sind Mittel, die zusätzlich zu den erfindungswesentlichen reaktiven Carbonylverbindungen zwingend 4-Hydroxy-2-methoxybenzaldehyd 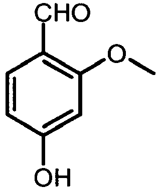 enthalten.
Weiterhin besonders bevorzugt sind Mittel, die weiterhin 2-(Cyanmethyl)-benzimidazol 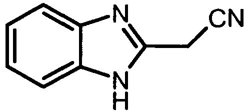 enthalten.

In einer weiteren Ausführungsform haben sich Mittel als besonders bevorzugt erwiesen, die mindestens eine weitere reaktive Carbonylverbindung ausgewählt aus 3,4-Dihydroxy-5-methoxybenzaldehyd, 2-Chlor-3,4-dihydroxybenzaldehyd und/oder 2-Brom-3,4-dihydroxybenzaldehyd enthalten.

Die erfindungsgemäßen Mittel enthalten die reaktiven Carbonylverbindungen vorzugsweise jeweils in einer Menge von 0,03 bis 65 mmol, insbesondere von 1 bis 40 mmol, bezogen auf 100 g des gesamten Färbemittels.

Die erfindungsgemäßen Mittel enthalten die C,H-aciden Verbindungen vorzugsweise in einer Menge von 0,03 bis 65 mmol, insbesondere von 1 bis 30 mmol, bezogen auf 100 g des gesamten Färbemittels, Auch die folgenden Kombinationen sind erfindungsgemäß bevorzugt:
- 1-Allyl-1,2-dihydro-3,4,6-trimethyl-2-oxo-pyrimidinium-bromid + 2,3-Dihydro-1,5-dimethyl-3-oxo-2-phenyl-1H-pyrazol-4-carboxaldehyd
- 1,2-Dihydro-1,3,4,6-tetramethyl-2-oxo-pyrimidimium-hydrogensulfat + 2,3-Dihydro-1,5-dimethyl-3-oxo-2-phenyl-1H-pyrazol-4-carboxaldehyd
- 1-Allyl-1,2-dihydro-3,4,6-trimethyl-2-oxo-pyrimidinium-bromid + 1,2-Dihydro-1,3,4,6-tetramethyl-2-oxo-pyrimidimium-hydrogensulfat + 2,3-Dihydro-1,5-dimethyl-3-oxo-2-phenyl-1 H-pyrazol-4-carboxaldehyd
- 1-Allyl-1,2-dihydro-3,4,6-trimethyl-2-oxo-pyrimidinium-bromid + 2,3-Dihydro-1,5-dimethyl-3-oxo-2-phenyl-1 H-pyrazol-4-carboxaldehyd + 2,4-Dimethoxy-benzaldehyd
- 1,2-Dihydro-1,3,4,6-tetramethyl-2-oxo-pyrimidimium-hydrogensulfat + 2,3-Dihydro-1,5-dimethyl-3-oxo-2-phenyl-1H-pyrazol-4-carboxaldehyd + 2,4-Dimethoxy-benzaldehyd
- 1-Allyl-1,2-dihydro-3,4,6-trimethyl-2-oxo-pyrimidinium-bromid + 1,2-Dihydro-1,3,4,6-tetramethyl-2-oxo-pyrimidimium-hydrogensulfat + 2,3-Dihydro-1,5-dimethyl-3-oxo-2-phenyl-1H-pyrazol-4-carboxaldehyd + 2,4-Dimethoxy-benzaldehyd
- 1-Allyl-1,2-dihydro-3,4,6-trimethyl-2-oxo-pyrimidinium-bromid + 2,3-Dihydro-1,5-dimethyl-3-oxo-2-phenyl-1H-pyrazol-4-carboxaldehyd + 4-Hydroxy-2-methoxybenzaldehyd
- 1,2-Dihydro-1,3,4,6-tetramethyl-2-oxo-pyrimidimium-hydrogensulfat + 2,3-Dihydro-1,5-dimethyl-3-oxo-2-phenyl-1 H-pyrazol-4-carboxaldehyd + 4-Hydroxy-2-methoxybenzaldehyd
- 1-Allyl-1,2-dihydro-3,4,6-trimethyl-2-oxo-pyrimidinium-bromid + 1,2-Dihydro-1,3,4,6-tetramethyl-2-oxo-pyrimidimium-hydrogensulfat + 2,3-Dihydro-1,5-dimethyl-3-oxo-2-phenyl-1H-pyrazol-4-carboxaldehyd + 4-Hydroxy-2-methoxybenzaldehyd
- 1-Allyl-1,2-dihydro-3,4,6-trimethyl-2-oxo-pyrimidinium-bromid + 2,3-Dihydro-1,5-dimethyl-3-oxo-2-phenyl-1 H-pyrazol-4-carboxaldehyd + 2,4-Dimethoxy-benzaldehyd + 4-Hydroxy-2-methoxybenzaldehyd
- 1,2-Dihydro-1,3,4,6-tetramethyl-2-oxo-pyrimidimium-hydrogensulfat + 2,3-Dihydro-1,5-dimethyl-3-oxo-2-phenyl-1 H-pyrazol-4-carboxaldehyd + 2,4-Dimethoxy-benzaldehyd + 4-Hydroxy-2-methoxybenzaldehyd
- 1-Allyl-1,2-dihydro-3,4,6-trimethyl-2-oxo-pyrimidinium-bromid + 1,2-Dihydro-1,3,4,6-tetramethyl-2-oxo-pyrimidimium-hydrogensulfat + 2,3-Dihydro-1,5-dimethyl-3-oxo-2-phenyl-1H-pyrazol-4-carboxaldehyd + 2,4-Dimethoxy-benzaldehyd + 4-Hydroxy-2-methoxybenzaldehyd
- 1-Allyl-1,2-dihydro-3,4,6-trimethyl-2-oxo-pyrimidinium-bromid + 2,3-Dihydro-1,5-dimethyl-3-oxo-2-phenyl-1 H-pyrazol-4-carboxaldehyd + 2-(Cyanmethyl)-benzimidazol
- 1,2-Dihydro-1,3,4,6-tetramethyl-2-oxo-pyrimidimium-hydrogensulfat + 2,3-Dihydro-1,5-dimethyl-3-oxo-2-phenyl-1H-pyrazol-4-carboxaldehyd + 2-(Cyanmethyl)-benzimidazol
- 1-Allyl-1,2-dihydro-3,4,6-trimethyl-2-oxo-pyrimidinium-bromid + 1,2-Dihydro-1,3,4,6-tetramethyl-2-oxo-pyrimidimium-hydrogensulfat + 2,3-Dihydro-1,5-dimethyl-3-oxo-2-phenyl-1H-pyrazol-4-carboxaldehyd + 2-(Cyanmethyl)-benzimidazol
- 1-Allyl-1,2-dihydro-3,4,6-trimethyl-2-oxo-pyrimidinium-bromid + 2,3-Dihydro-1,5-dimethyl-3-oxo-2-phenyl-1 H-pyrazol-4-carboxaldehyd + 2,4-Dimethoxy-benzaldehyd + 2-(Cyanmethyl)-benzimidazol
- 1,2-Dihydro-1,3,4,6-tetramethyl-2-oxo-pyrimidimium-hydrogensulfat + 2,3-Dihydro-1,5-dimethyl-3-oxo-2-phenyl-1 H-pyrazol-4-carboxaldehyd + 2,4-Dimethoxy-benzaldehyd + 2-(Cyanmethyl)-benzimidazol
- 1-Allyl-1,2-dihydro-3,4,6-trimethyl-2-oxo-pyrimidinium-bromid + 1,2-Dihydro-1,3,4,6-tetramethyl-2-oxo-pyrimidimium-hydrogensulfat + 2,3-Dihydro-1,5-dimethyl-3-oxo-2-phenyl-1H-pyrazol-4-carboxaldehyd + 2,4-Dimethoxy-benzaldehyd + 2-(Cyanmethyl)-benzimidazol
- 1-Allyl-1,2-dihydro-3,4,6-trimethyl-2-oxo-pyrimidinium-bromid + 2,3-Dihydro-1,5-dimethyl-3-oxo-2-phenyl-1H-pyrazol-4-carboxaldehyd + 4-Hydroxy-2-methoxybenzaldehyd + 2-(Cyanmethyl)-benzimidazol
- 1,2-Dihydro-1,3,4,6-tetramethyl-2-oxo-pyrimidimium-hydrogensulfat + 2,3-Dihydro-1,5-dimethyl-3-oxo-2-phenyl-1H-pyrazol-4-carboxaldehyd + 4-Hydroxy-2-methoxybenzaldehyd + 2-(Cyanmethyl)-benzimidazol
- 1-Allyl-1,2-dihydro-3,4,6-trimethyl-2-oxo-pyrimidinium-bromid + 1,2-Dihydro-1,3,4,6-tetramethyl-2-oxo-pyrimidimium-hydrogensulfat + 2,3-Dihydro-1,5-dimethyl-3-oxo-2-phenyl-1H-pyrazol-4-carboxaldehyd + 4-Hydroxy-2-methoxybenzaldehyd + 2-(Cyanmethyl)-benzimidazol
- 1-Allyl-1,2-dihydro-3,4,6-trimethyl-2-oxo-pyrimidinium-bromid + 2,3-Dihydro-1,5-dimethyl-3-oxo-2-phenyl-1H-pyrazol-4-carboxaldehyd + 2,4-Dimethoxy-benzaldehyd + 4-Hydroxy-2-methoxybenzaldehyd + 2-(Cyanmethyl)-benzimidazol
- 1,2-Dihydro-1,3,4,6-tetramethyl-2-oxo-pyrimidimium-hydrogensulfat + 2,3-Dihydro-1,5-dimethyl-3-oxo-2-phenyl-1 H-pyrazol-4-carboxaldehyd + 2,4-Dimethoxy-benzaldehyd + 4-Hydroxy-2-methoxybenzaldehyd + 2-(Cyanmethyl)-benzimidazol
- 1-Allyl-1,2-dihydro-3,4,6-trimethyl-2-oxo-pyrimidinium-bromid + 1,2-Dihydro-1,3,4,6-tetramethyl-2-oxo-pyrimidimium-hydrogensulfat + 2,3-Dihydro-1,5-dimethyl-3-oxo-2-phenyl-1H-pyrazol-4-carboxaldehyd + 2,4-Dimethoxy-benzaldehyd + 4-Hydroxy-2-methoxybenzaldehyd + 2-(Cyanmethyl)-benzimidazol

Im Rahmen einer bevorzugten Ausführungsform liegt das erfindungsgemäße Färbemittel in Form eines Kits (Verpackungseinheit) mit folgenden getrennt voneinander konfektionierten Komponenten vor:
- Mittel A1, das mindestens eine der besagten C,H-aciden Verbindungen 1-Allyl-1,2-dihydro-3,4,6-trimethyl-2-oxo-pyrimidinium, 1,2-Dihydro-1,3,4,6-tetramethyl-2-oxo-pyrimidimium und gegebenenfalls 2-(Cyanmethyl)-benzimidazol in einem flüssigen kosmetischen Träger enthält und einen pH-Wert von 0,5 bis 2,5 aufweist und
- Mittel A2, das mindestens eine der besagten reaktiven Carbonylverbindungen 2,3-Dihydro-1,5-dimethyl-3-oxo-2-phenyl-1H-pyrazol-4-carboxaldehyd und/oder 2,4-Dimethoxy-benzaldehyd sowie gegebenenfalls 4-Hydroxy-2-methoxybenzaldehyd, 3,4-Dihydroxy-5-methoxybenzaldehyd, 2-Chlor-3,4-dihydroxybenzaldehyd und/oder 2-Brom-3,4-dihydroxybenzaldehyd in einem flüssigen kosmetischen Träger enthält und einen pH-Wert von 2,5 bis 5 aufweist.

Besonders lagerstabile Formulierungen können erhalten werden, wenn das Mittel A1 einen pH-Wert zwischen 1,1 und 1,9 aufweist, daher ist dieser pH-Bereich für das Mittel A1 bevorzugt.

Des Weiteren ist es bevorzugt, wenn das Mittel A2 einen pH-Wert von 2,5 bis 5 und insbesondere bevorzugt einen pH-Wert von 3,2 bis 4,5 aufweist.

Bei den pH-Werten im Sinne der vorliegenden Erfindung handelt es sich um pH-Werte, die bei einer Temperatur von 22 °C gemessen wurden.

Unter flüssig wird erfindungsgemäß verstanden, wenn der betrachtete Stoff / das Stoffgemisch bei 25°C bei einem Druck von 1 atm einen flüssigen Aggregatzustand besitzt.

Die Einstellung des pH-Wertes in den Mitteln A1 und/oder A2 kann mit Hilfe einer organischen oder anorganischen Säure wie beispielsweise Salzsäure, Schwefelsäure, Bromwasserstoffsäure, Phosphorsäure, Essigsäure, Weinsäure, Zitronensäure, Milchsäure, Äpfelsäure oder Glykolsäure erfolgen.

In diesem Zusammenhang ist die Einstellung der erfindungsgemäßen pH-Werte mit Hilfe von Schwefelsäure, Salzsäure, Weinsäure, Zitronensäure, Äpfelsäure oder Milchsäure besonders bevorzugt. Für die Anwendung des erfindungsgemäßen Kits können die Mittel A1 und A2 kurz vor dem Aufbringen auf die Haarfaser innig miteinander vermischt werden. Zur weiteren Verbesserung des Färbeergebnisses kann es jedoch vorteilhaft sein, die Färbung selbst in einem alkalischen pH-Bereich durchzuführen. Daher kann im Rahmen einer besonderen Ausführungsform das erfindungsgemäße Kit zusätzlich ein drittes, kosmetisches Mittel A3 umfassen, welches in einem kosmetischen Träger mindestens ein Alkalisierungsmittel enthält und einen pH-Wert von 7 bis 10 aufweist.

Erfindungsgemäß bevorzugt sind somit solche Kits, in denen die Mittel A1, A2 und A3 so ausgestaltet sind, dass das durch Vermischen der Komponenten resultierende anwendungsbereite Färbemittel einen pH-Wert von 7 bis 10 aufweist. Diese kosmetischen Mittel A3 sind bevorzugt flüssig.

Die kosmetischen Mittel A3 enthalten bevorzugt in einem kosmetischen Träger mindestens ein Alkalisierungsmittel.

Die im kosmetischen Mittel A3 enthaltenen Alkalisierungsmittel werden bevorzugt aus mindestens einem Alkalisierungsmittel ausgewählt aus der Gruppe, die gebildet wird, aus Ammoniak, basischen Aminosäuren, Alkalihydroxiden, Alkanolaminen, Alkalimetallmetasilikaten, Harnstoff, Morpholin, N-Methylglucamin, Imidazol, Alkaliphosphaten und Alkalihydrogenphosphaten. Als Alkalimetallionen dienen bevorzugt Lithium, Natrium, Kalium, insbesondere Natrium oder Kalium.

Die als erfindungsgemäßes Alkalisierungsmittel einsetzbaren basischen Aminosäuren werden bevorzugt ausgewählt aus der Gruppe, die gebildet wird aus L-Arginin, D-Arginin, D,L-Arginin, L-Histidin, D-Histidin, D,L-Histidin, L-Lysin, D-Lysin, D,L-Lysin, besonders bevorzugt L-Arginin, D-Arginin, D,L-Arginin als ein Alkalisierungsmittel im Sinne der Erfindung eingesetzt.

Die als erfindungsgemäßes Alkalisierungsmittel einsetzbaren Alkalihydroxide werden bevorzugt ausgewählt aus der Gruppe, die gebildet wird aus Natriumhydroxid und Kaliumhydroxid.

Die als erfindungsgemäßes Alkalisierungsmittel einsetzbaren Alkanolamine werden bevorzugt ausgewählt aus primären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine werden ausgewählt aus der Gruppe 2-Aminoethan-1-ol, 2-Amino-2-methylpropan-1-ol und 2-Amino-2-methyl-propan-1,3-diol.

Besonders bevorzugt wird das Alkalisierungsmittel ausgewählt aus mindestens einer Verbindung aus der Gruppe, die gebildet wird aus Ammoniak, 2-Aminoethanol, 2-Amino-2-methylpropan-1-ol, 2-Amino-2-methyl-propan-1,3-diol, Kaliumhydroxid, Natriumhydroxid, L-Arginin, D-Arginin, DL-Arginin, N-Methylglucamin, Morpholin und Harnstoff.

Der pH-Wert des anwendungsbereiten Färbemittels, welches durch Vermischen der Mittel A1, A2 und A3, hergestellt wird, liegt bevorzugt zwischen 7,5 und 11. Liegt der pH-Wert des besagten anwendungsbereiten Färbemittels in einem Bereich von 7,5 bis 9,0, so ist dies bevorzugt.

In einer weiteren Ausführungsform kann das erfindungsgemäße Mittel zusätzlich mindestens eine Entwicklerkomponente und gegebenenfalls mindestens eine Kupplerkomponente als Oxidationsfarbstoffvorprodukte enthalten. Es ist jedoch bevorzugt die erfindungsgemäßen Mittel frei von Oxidationsfarbstoffvorprodukten zu formulieren, insbesondere wenn das Allergierisiko für para-Allergiker minimiert werden soll. Somit kennzeichnet sich ein bevorzugtes erfindungsgemäßes Mittel dadurch, dass es frei von Oxidationsfarbstoffvorprodukten vom Entwicklertyp sind.

Es kann erfindungsgemäß bevorzugt sein, als Entwicklerkomponente ein p-Phenylendiaminderivat oder eines seiner physiologisch verträglichen Salze einzusetzen. Erfindungsgemäß ganz besonders bevorzugte p-Phenylendiaminderivate sind ausgewählt aus mindestens einer Verbindung der Gruppe p-Phenylendiamin, p-Toluylendiamin, 2-(β-Hydroxyethyl)-p-phenylendiamin, 2-(α,β-Dihydroxyethyl)-p-phenylendiamin, N,N-Bis-(β-hydroxyethyl)-p-phenylendiamin, N-(4-Amino-3-methylphenyl)-N-[3-(1H-imidazol-1-yl)propyl]amin, sowie den physiologisch verträglichen Salzen dieser Verbindungen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind. Ganz besonders bevorzugte zweikernige Entwicklerkomponenten werden ausgewählt unter N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan oder eines der physiologisch verträglichen Salze dieser Verbindungen.

Weiterhin kann es erfindungsgemäß bevorzugt sein, als Entwicklerkomponente ein p-Aminophenolderivat oder eines seiner physiologisch verträglichen Salze einzusetzen. Ganz besonders bevorzugte

Verbindungen sind p-Aminophenol, 4-Amino-3-methylphenol, 4-Amino-2-aminomethylphenol, 4-Amino-2-(α,β-dihydroxyethyl)-phenol und 4-Amino-2-(diethylaminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol. Weiterhin kann die Entwicklerkomponente ausgewählt sein aus heterocyclischen Entwicklerkomponenten, wie beispielsweise aus Pyrimidinderivaten, Pyrazolderivaten, Pyrazolopyrimidin-Derivaten bzw. ihren physiologisch verträglichen Salzen.

Besonders bevorzugte Pyrimidin-Derivate sind insbesondere die Verbindungen 2,4,5,6-Tetraamino-pyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Besonders bevorzugte Pyrazol-Derivate sind insbesondere die Verbindungen, die ausgewählt werden unter 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)amino-1,3-dimethylpyrazol, sowie deren physiologisch verträglichen Salze.

Ganz besonders bevorzugte Entwicklerkomponenten werden ausgewählt, aus mindestens einer Verbindung aus der Gruppe, die gebildet wird aus p-Phenylendiamin, p-Toluylendiamin, 2-(β-Hydroxyethyl)-p-phenylendiamin, 2-(α,β-Dihydroxyethyl)-p-phenylendiamin, N,N-Bis-(β-hydroxyethyl)-p-phenylendiamin, N-(4-Amino-3-methylphenyl)-N-[3-(1H-imidazol-1-yl)propyl]amin, N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(α,β-dihydroxyethyl)-phenol und 4-Amino-2-(diethylaminomethyl)-phenol, 4,5-Diamino-1-(β-hydroxyethyl)-pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen. Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus. Kuppler sind bevorzugt zyklische Verbindungen, die am Zyklus mindestens zwei Gruppen tragen, ausgewählt aus (i) gegebenenfalls substituierten Aminogruppen und/oder (ii) Hydroxygruppen. Wenn die zyklische Verbindung ein Sechsring (bevorzugt aromatisch) ist, so befinden sich die besagten Gruppen bevorzugt in ortho-Position oder meta-Position zueinander.

Erfindungsgemäße Kupplerkomponenten werden bevorzugt als mindestens eine Verbindung aus einer der folgenden Klassen ausgewählt:
- m-Aminophenol und/oder dessen Derivate,
- m-Diaminobenzol und/oder dessen Derivate,
- o-Diaminobenzol und/oder dessen Derivate,
- o-Aminophenolderivate, wie beispielsweise o-Aminophenol,
- Naphthalinderivate mit mindestens einer Hydroxygruppe,
- Di- beziehungsweise Trihydroxybenzol und/oder deren Derivate,
- Pyridinderivate,
- Pyrimidinderivate,
- Monohydroxyindol-Derivate und/oder Monoaminoindol-Derivate,
- Monohydroxyindolin-Derivate und/oder Monoaminoindolin-Derivate,
- Pyrazolonderivate, wie beispielsweise 1-Phenyl-3-methylpyrazol-5-on,
- Morpholinderivate wie beispielsweise 6-Hydroxybenzomorpholin oder 6-Amino-benzomorpholin,
- Chinoxalinderivate wie beispielsweise 6-Methyl-1,2,3,4-tetrahydrochinoxalin,

Gemische aus zwei oder mehrer Verbindungen aus einer oder mehrerer dieser Klassen sind im Rahmen dieser Ausführungsform ebenso erfindungsgemäß.

Besonders bevorzugte m-Aminophenol-Kupplerkomponenten werden ausgewählt aus mindestens einer Verbindung aus der Gruppe, die gebildet wird aus m-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol, 2,4-Dichlor-3-aminophenol und den physiologisch verträglichen Salzen aller vorstehend genannten Verbindungen.

Besonders bevorzugte m-Diaminobenzol-Kupplerkomponenten werden ausgewählt aus mindestens einer Verbindung aus der Gruppe, die gebildet wird aus m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)-aminobenzol und den physiologisch verträglichen Salzen aller vorstehend genannten Verbindungen.

Besonders bevorzugte o-Diaminobenzol-Kupplerkomponenten werden ausgewählt aus mindestens einer Verbindung aus der Gruppe, die gebildet wird aus 3,4-Diaminobenzoesäure und 2,3-Diamino-1-methylbenzol und den physiologisch verträglichen Salzen aller vorstehend genannten Verbindungen. Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Besonders bevorzugte Pyridinderivate werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und den physiologisch verträglichen Salzen der vorgenannten Verbindungen.

Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Besonders bevorzugte Indolderivate werden ausgewählt aus mindestens einer Verbindung der Gruppe, die gebildet wird aus 4-Hydroxyindol, 6-Hydroxyindol und 7-Hydroxyindol und den physiologisch verträglichen Salzen der vorgenannten Verbindungen.

Besonders bevorzugte Indolinderivate werden ausgewählt aus mindestens einer Verbindung der Gruppe, die gebildet wird aus 4-Hydroxyindolin, 6-Hydroxyindolin und 7-Hydroxyindolin und den physiologisch verträglichen Salzen der vorgenannten Verbindungen.

Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und den physiologisch verträglichen Salzen der vorgenannten Verbindungen.

Erfindungsgemäß besonders bevorzugte Kupplerkomponenten werden ausgewählt unter m-Aminophenol, 5-Amino-2-methylphenol, 3-Amino-2-chlor-6-methylphenol, 2-Hydroxy-4-amino-phenoxyethanol, 5-Amino-4-chlor-2-methylphenol, 5-(2'-Hydroxyethyl)-amino-2-methylphenol, 2,4-Dichlor-3-aminophenol, o-Aminophenol, m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methyl-phenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)-aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen.

Die Kupplerkomponenten werden bevorzugt in einer Menge von 0,005 bis 20 Gew.-%, vorzugsweise 0,1 bis 5 Gew.-%, jeweils bezogen auf das anwendungsbereite Gemisch aus Mittel A, Mittel B und gegebenenfalls Mittel C, verwendet.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, stehen können.

Es kann erfindungsgemäß bevorzugt sein, dem erfindungsgemäßen Mittel als weitere Farbstoffvorstufe einen naturanalogen Farbstoff zuzusetzen.

Als Farbstoffvorstufen naturanaloger Farbstoffe werden bevorzugt solche Indole und Indoline eingesetzt, die mindestens zwei Gruppen ausgewählt aus Hydroxy- und/oder oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer weiteren Ausführungsform enthalten die Färbemittel mindestens ein Indol- und/oder Indolinderivat. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt.

Besonders gut als Vorstufen naturanaloger Haarfarbstoffe geeignet sind Derivate des 5,6-Dihydroxyindolins. Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxy-indolin und insbesondere das 5,6-Dihydroxyindolin.

Als Vorstufen naturanaloger Haarfarbstoffe hervorragend geeignet sind weiterhin Derivate des 5,6-Dihydroxyindols. Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- beziehungsweise die Indol-Derivate können sowohl als freie Basen als auch in Form ihrer physiologisch verträglichen Salze mit anorganischen oder organischen Säuren, z. B. der Hydrochloride, der Sulfate und Hydrobromide, eingesetzt werden.

Weiterhin kann in dem erfindungsgemäßen Mittel zusätzlich mindestens ein direktziehender Farbstoff enthalten sein. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Die direktziehenden Farbstoffe werden jeweils bevorzugt in einer Menge von 0,001 bis 20 Gew.-%, bezogen auf die gesamte Anwendungszubereitung, eingesetzt. Die Gesamtmenge an direktziehenden Farbstoffen beträgt vorzugsweise höchstens 20 Gew.-%.

Direktziehende Farbstoffe können in anionische, kationische und nichtionische direktziehende Farbstoffe unterteilt werden.

### Anionische direktziehende Farbstoffe:

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

### Kationische direktziehende Farbstoffe:

Bevorzugte kationische direktziehenden Farbstoffe sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Die unter den Bezeichnungen Basic Yellow 87, Basic Orange 31 und Basic Red 51 bekannten Verbindungen sind ganz besonders bevorzugte kationische direktziehende Farbstoffe der Gruppe (c). Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe. Nichtionische direktziehende Farbstoffe:
Als nichtionische direktziehende Farbstoffe eignen sich insbesondere nichtionische Nitro- und Chinonfarbstoffe und neutrale Azofarbstoffe.

Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, dass die direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind.

Die erfindungsgemäßen Mittel können die fakultativ enthaltenen Entwickler, Kuppler und/oder Direktzieher in einer Menge von 0,01 bis 20,00 Gew.-%, bezogen auf den erfindungsgemäßen Kit und damit bezogen auf eine Mischung aus Mittel A und Mittel B und gegebenenfalls zusätzlich Mittel C enthalten.
- Weiterhin kann der erfindungsgemäße Kit auch in der Natur vorkommende Farbstoffe, wie sie beispielsweise in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten sind, enthalten.

Zur Erlangung weiterer und intensiverer Ausfärbungen das erfindungsgemäße Mittel zusätzlich Farbverstärker enthalten. Die Farbverstärker sind vorzugsweise ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Arginin, Histidin, Pyrrolidin, Prolin, Pyrrolidon, Pyrrolidon-5-carbonsäure, Pyrazol, 1,2,4-Triazol, Piperazidin, deren Derivate sowie deren physiologisch verträglichen Salzen.

Die voran stehend genannten Farbverstärker können in einer Menge von jeweils 0,03 bis 65 mmol, insbesondere 1 bis 40 mmol, jeweils bezogen auf 100 g der fertigen Anwendungsmischung, eingesetzt werden.

Auf die Anwesenheit von Oxidationsmitteln, z. B. H₂O₂, kann, insbesondere wenn das erfindungsgemäße Mittel keine Oxidationsfarbstoffvorprodukte enthält, verzichtet werden. Wenn das erfindungsgemäße Mittel luftoxidable Oxidationsfarbstoffvorprodukte oder Indol bzw. Indolinderivate enthält, kann in einem solchen Fall ohne Probleme auf Oxidationsmittel verzichtet werden. Es kann jedoch unter Umständen wünschenswert sein, dem erfindungsgemäßen Kit zur Erzielung der Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die fertige Anwendungsmischung, eingesetzt. Ein für menschliches Haar bevorzugtes Oxidationsmittel ist H₂O₂. Auch Gemische von mehreren Oxidationsmitteln, wie beispielsweise eine Kombination aus Wasserstoffperoxid und Peroxodisulfaten der Alkali- und Erdalkalimetalle oder aus Iodidionenquellen, wie z.B. Alkalimetalliodiden und Wasserstoffperoxid oder den vorgenannten Peroxodisulfaten, können verwendet werden. Das Oxidationsmittel bzw. die Oxidationsmittelkombination können erfindungsgemäß in Verbindung mit Oxidationskatalysatoren in dem Haarfärbemittel zur Anwendung kommen. Oxidationskatalysatoren sind beispielsweise Metallsalze, Metallchelat-Komplexe oder Metalloxide, die einen leichten Wechsel zwischen zwei Oxidationsstufen der Metallionen ermöglichen. Beispiele sind Salze, Chelatkomplexe oder Oxide von Eisen, Ruthenium, Mangan und Kupfer. Weitere mögliche Oxidationskatalysatoren stellen Enzyme dar. Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten.

Weiterhin kann das erfindungsgemäße Kit alle in solchen Zubereitungen bekannten Wirk-, Zusatz- und Hilfsstoffe enthalten. Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butyl-maleat/Isobornylacrylat-Copolymere, Methylvinylether/Maleinsäureanhydrid-Copolymere und Acrylsäure/Ethylacrylat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Glucose und Maleinsäure,
- Parfümöle, Dimethylisosorbid und Cyclodextrine,
- Antischuppenwirkstoffe wie Piroctone Olamine und Zink Omadine,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des kosmetischen Trägers werden zur Herstellung des erfindungsgemäßen Mittels in für diesen Zweck üblichen Mengen eingesetzt; z. B. werden Emulgiermittel in Konzentrationen von 0,5 bis 30 Gew.-% und Verdickungsmittel in Konzentrationen von 0,1 bis 25 Gew.-% bezogen auf das jeweilige Mittel eingesetzt.

Ein zweiter Gegenstand der vorliegenden Erfindung ist die Verwendung von Kombinationen aus C,H-aciden Verbindungen und reaktiven Carbonylverbindungen gemäß den Ansprüchen 1 bis 7 als färbende Komponente in Haarfärbemitteln.

Ein dritter Gegenstand der vorliegenden Erfindung ist ein Verfahren zum Färben von keratinhaltigen Fasern, insbesondere menschlichen Haaren, worin ein Färbemittel, gemäß einem der Ansprüche 1 bis 8, sowie übliche kosmetische Inhaltsstoffe, auf die keratinhaltigen Fasern aufgebracht, einige Zeit, üblicherweise ca. 15-30 Minuten, auf der Faser belassen und anschließend wieder ausgespült oder mit einem Shampoo ausgewaschen wird.

### Beispiele

Die in den Beispielen verwendeten Mengenangaben verstehen sich, sofern nicht anderes angegeben ist, in Gewichtsprozent.

### 1.1 Herstellung der Mittel (A1)

Es wurden die folgenden Mittel (A1), enthaltend mindestens eine C,H-acide Verbindung, hergestellt:

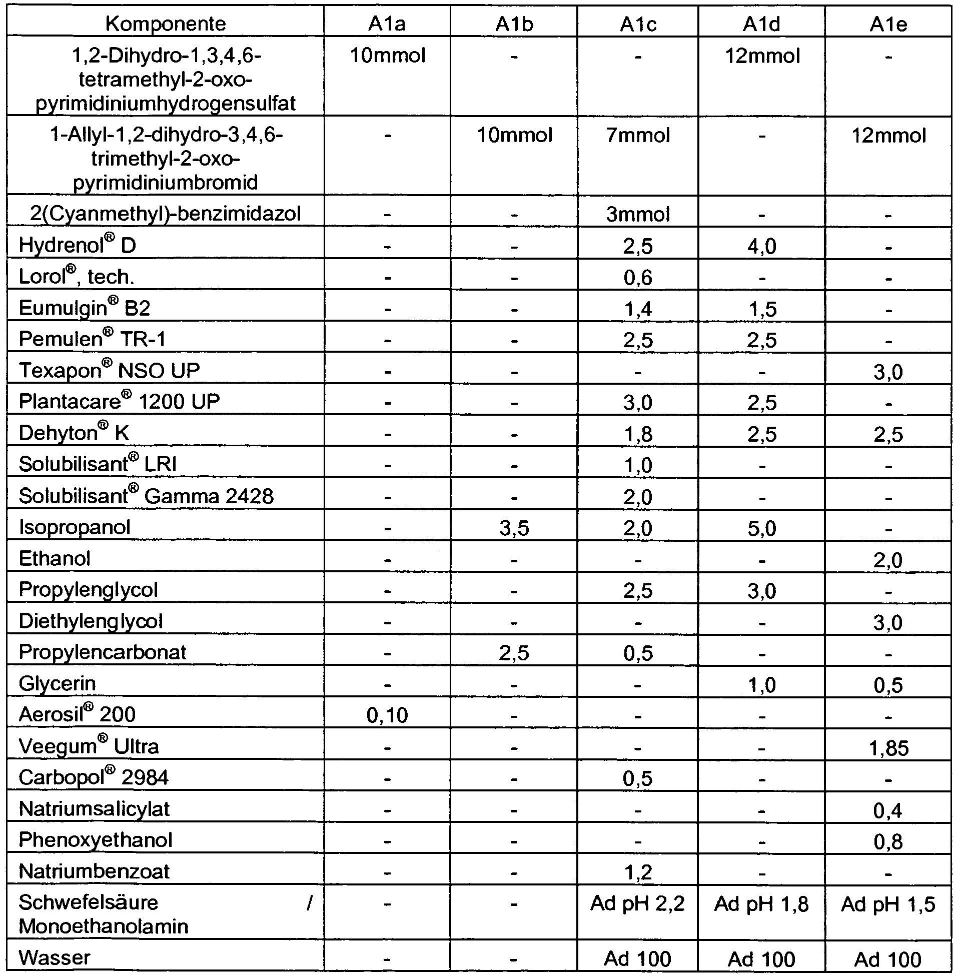

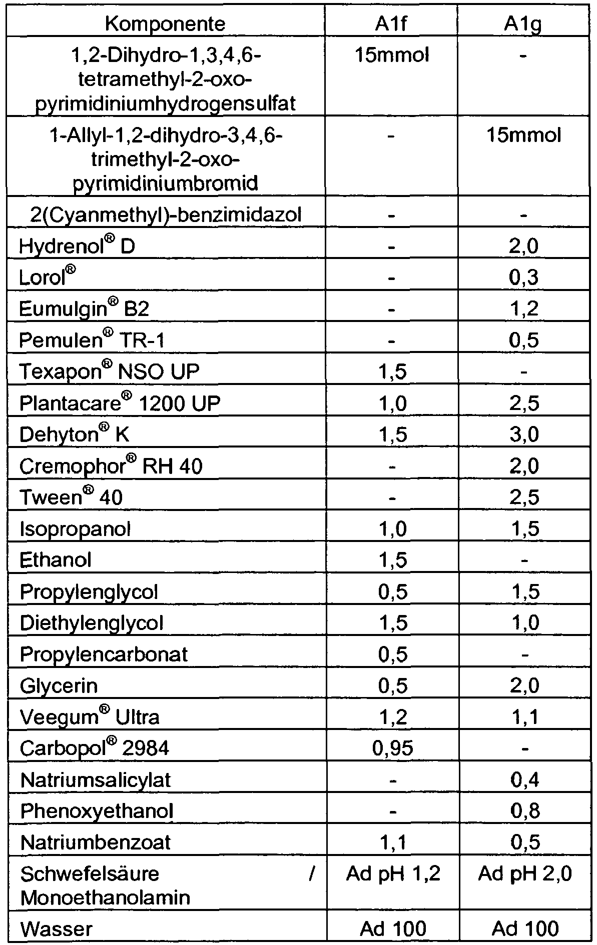

### 1.1.1 Herstellung der Cremeformulierungen A1c, A1d und A1g

Lorol^{®}, Eumulgin^{®} B2, Pemulen TR-1 und/oder Hydrenol^{®} D wurden zusammen bei 80°C aufgeschmolzen. In diese Schmelze wurden die für die Einzelformulierungen notwendigen Mengenanteile der Komponenten Texapon^{®} NSO UP, Dehyton^{®} K und/oder Plantacare^{®} 1200 UP eingearbeitet. 1-Allyl-1,2-dihydro-3,4,6-trimethyl-2-oxopyrimidiniumbromid, 1,2-Dihydro-1,3,4,6-tetramethyl-2-oxo-pyrimidinium-hydrogensulfonat und/oder 2-(Cyanmethyl)-benzimidazol gelöst in Isopropanol, Propylenglykol, Diethylenglykol, Propylencarbonat und/oder Glycerin mit 80 bis 90°C heißem Wasser, wurden unter Rühren hinzu gegeben. Nach dem Abkühlen wurden die übrigen Rezepturbestandteile, wie Cremophor^{®} RH 40, Tween^{®} 40, Solubilisant^{®} LRI, Solubilisant Gamma^{®} 2428, Natriumsalicylat, Phenoxyethanol, Natriumbenzoat und/oder Ethanol hinzu gegeben. Anschließend wurden Veegum^{®} Ultra und/oder Carbopol^{®} 2984 unter weiterem Rühren langsam hinzu gegeben. Der Quellvorgang wurde abgewartet. Durch die Zugabe von Schwefelsäure bzw. Monoethanolamin wurde der für die jeweilige Formulierung angegebene pH-Wert eingestellt. Zum Schluss wurde mit warmem Wasser auf 100g aufgefüllt.

### 1.1.2 Herstellung der Gelfomulierungen A1e und A1f

1-Allyl-1,2-dihydro-3,4,6-trimethyl-2-oxopyrimidiniumbromid, 1,2-Dihydro-1,3,4,6-tetramethyl-2-oxo-pyrimidinium-hydrogensulfonat und/oder 2-(Cyanmethyl)-benzimidazol wurden in70g Wasser, Isopropanol, Propylenglykol, Diethylenglykol, Propylencarbonat und/oder Glycerin unter Rühren gelöst, dann wurden die für die Einzelformulierungen notwendigen Mengenanteile der Komponenten Texapon^{®} NSO UP, Dehyton^{®} K und/oder Plantacare ^{®}1200 UP hinzu gegeben. Während des Abkühlens wurden die zuvor vorgequollenen Mittel Veegum^{®} Ultra und/oder Carbopol^{®} 2984 unter weiterem Rühren hinzu gegeben. Der Quellvorgang wurde abgewartet. Anschließend wurden die Mittel Natriumsalicylat, Phenoxyethanol und/oder Natriumbenzoat hinzugefügt. Zum Schluss wurde durch Zugabe von Schwefelsäure bzw. Monoethanolamin der für die jeweilige Formulierung angegebene pH-Wert eingestellt und mit kaltem Wasser auf 100g aufgefüllt.

### 1.2 Herstellung der Mittel (A2)

Es wurden die folgenden Mittel (A2), enthaltend mindestens eine reaktive Carbonylverbindung, hergestellt:

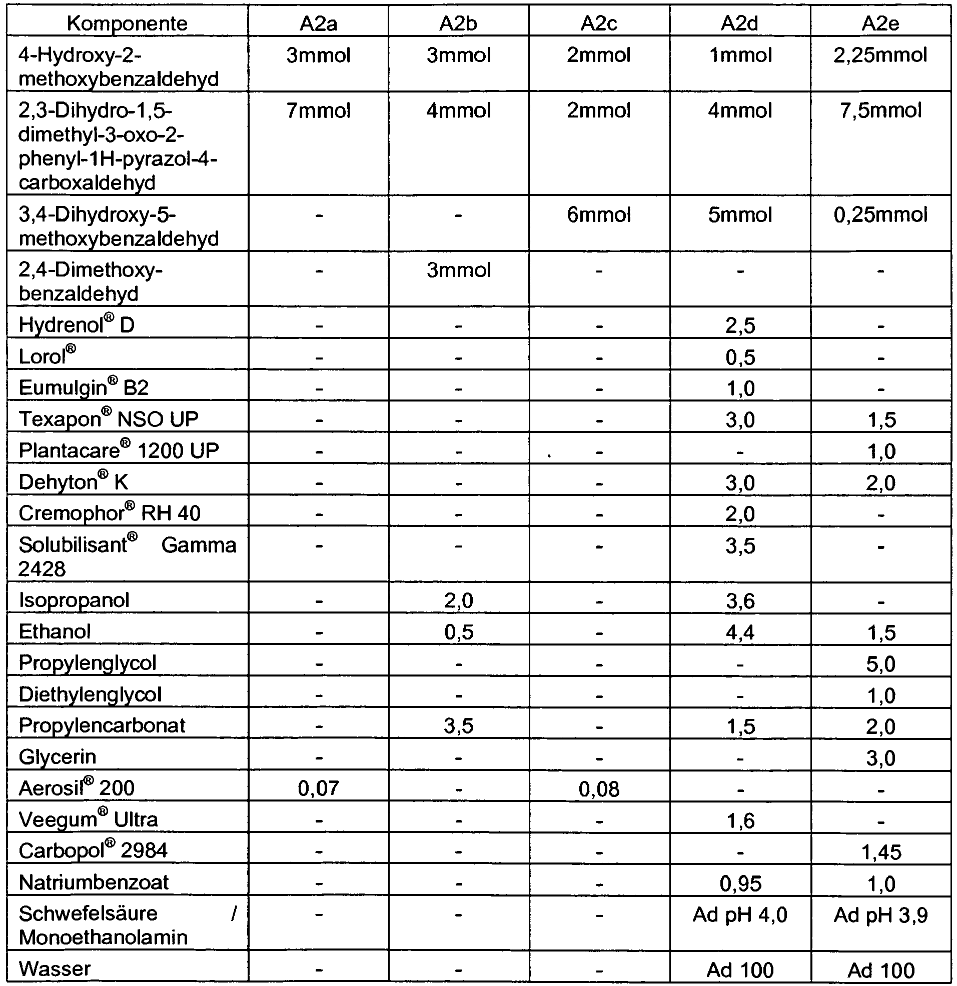

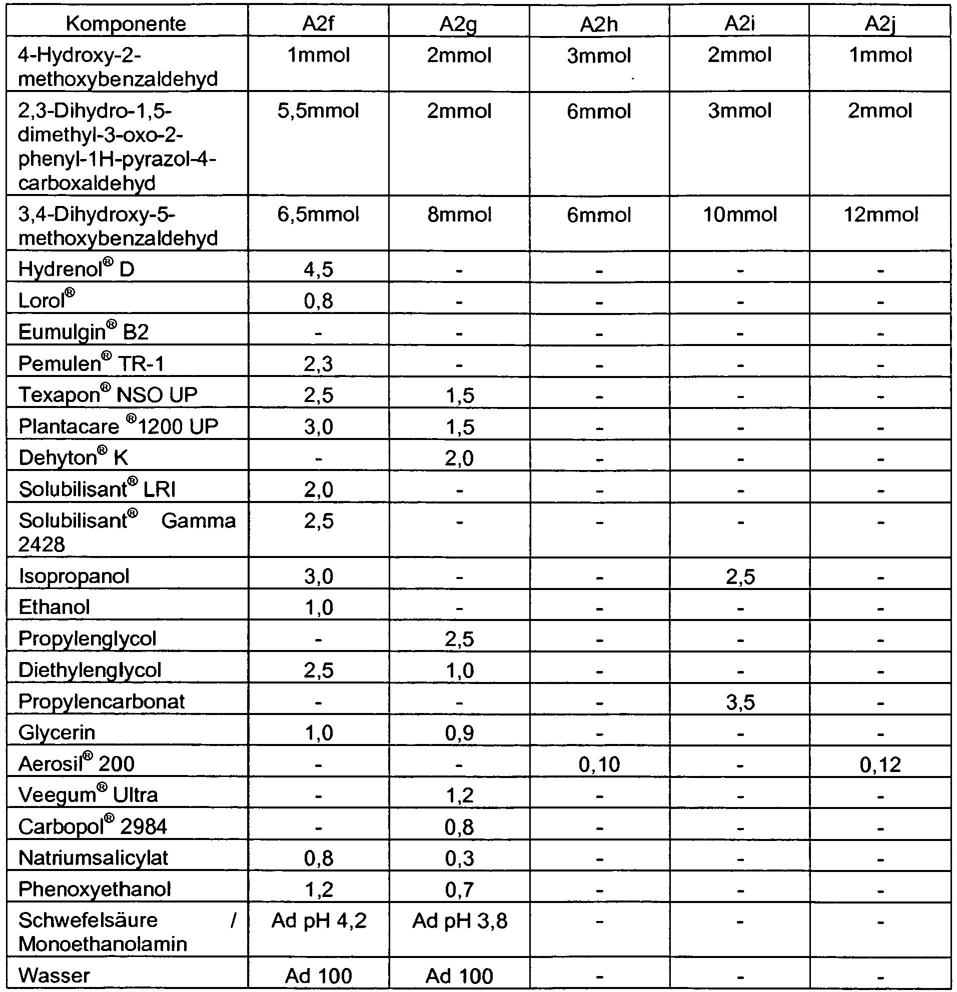

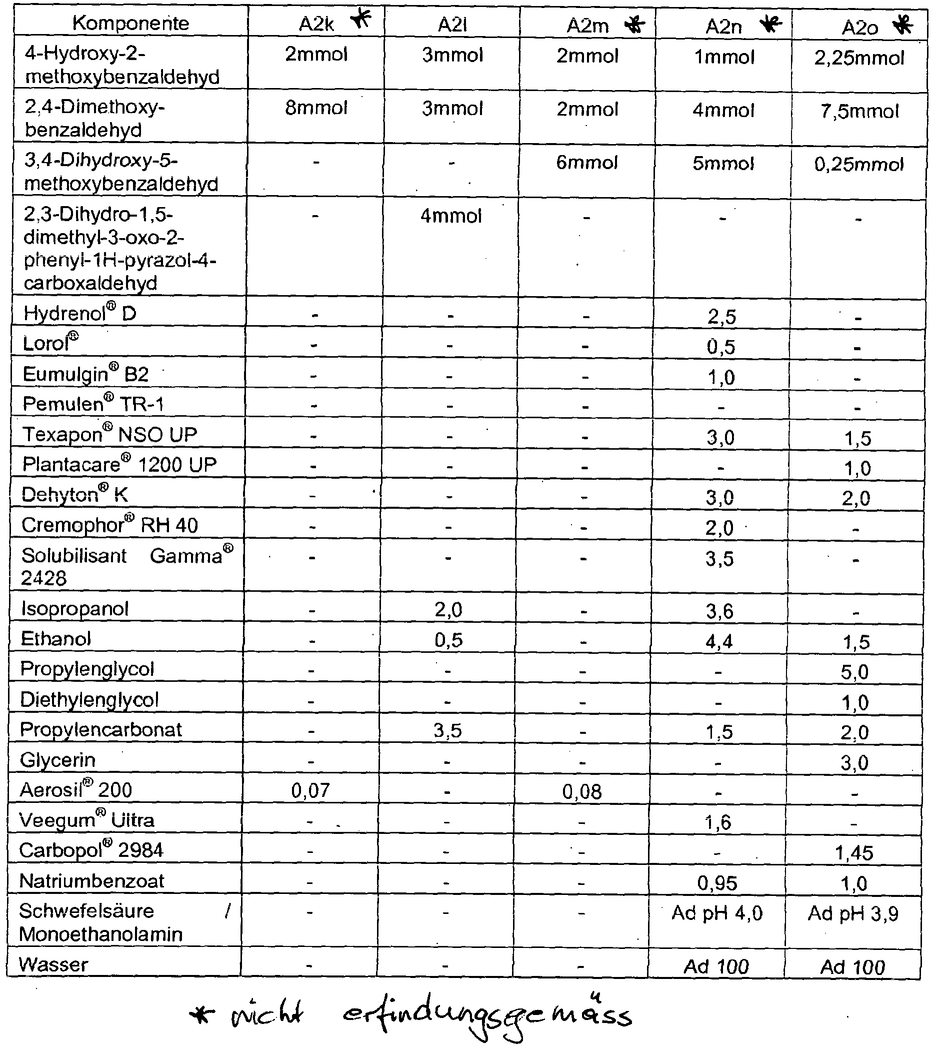

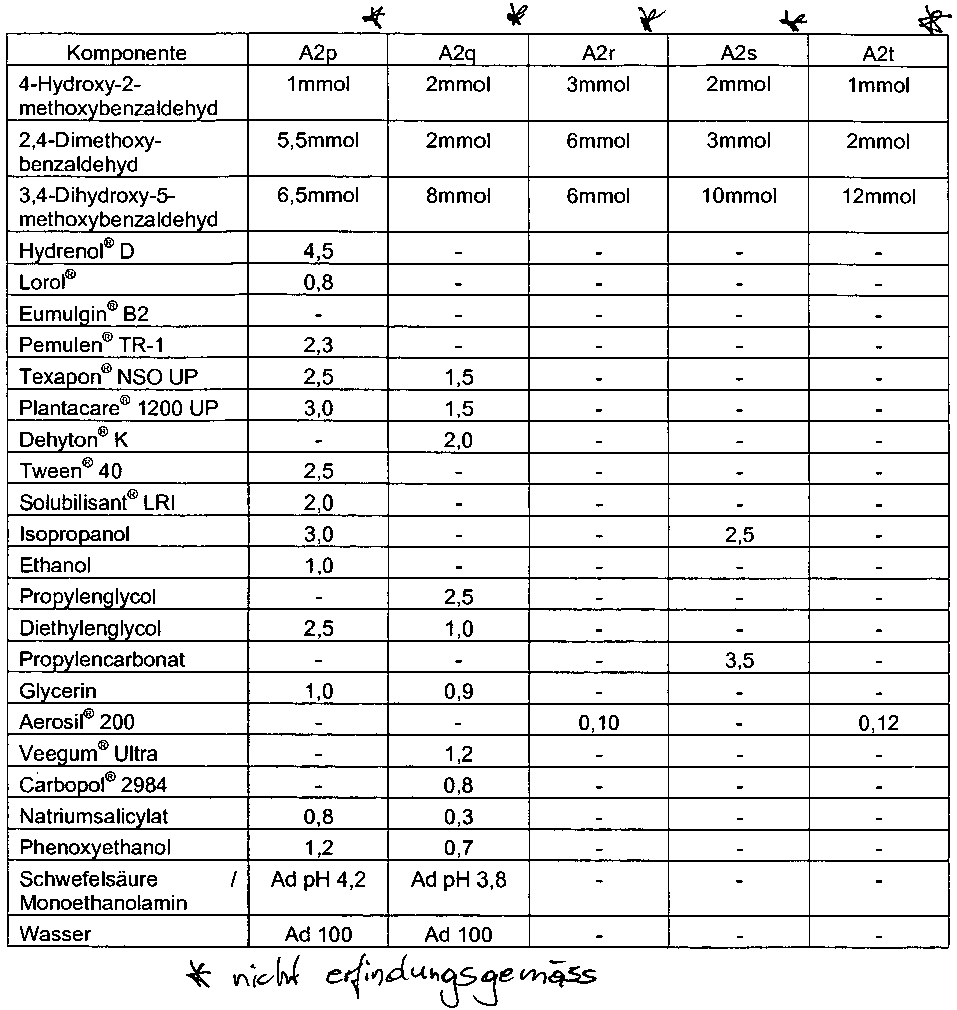

### 1.2.1 Herstellung der Cremeformulierungen A2d, A2f, A2n und A2p nicht erfindungsgemäss

Lorol^{®}, Eumulgin^{®} B2, Pemulen^{®} TR-1 und/oder Hydrenol^{®} D wurden zusammen bei 80°C aufgeschmolzen. In diese Schmelze wurden die für die Einzelformulierungen notwendigen Mengenanteile der Komponenten Texapon^{®} NSO UP, Dehyton^{®} K und/oder Plantacare^{®} 1200 UP eingearbeitet.

Die Aldehyde, gelöst in Isopropanol, Propylenglykol, Propylencarbonat und/oder Glycerin mit 80 bis 90°C heißem Wasser, wurden unter Rühren hinzu gegeben. Nach dem Abkühlen wurden die übrigen Rezepturbestandteile, wie Cremophor^{®} RH 40, Tween^{®} 40, Solubilisant^{®} LRI, Solubilisant Gamma^{®} 2428, Natriumsalicylat, Phenoxyethanol, Natriumbenzoat und/oder Ethanol hinzu gegeben. Anschließend wurden Veegum^{®} Ultra und/oder Carbopol^{®} 2984 unter weiterem Rühren langsam hinzu gegeben. Der Quellvorgang wurde abgewartet. Durch die Zugabe von Schwefelsäure bzw. Monoethanolamin wurde der für die jeweilige Formulierung angegebene pH-Wert eingestellt. Zum Schluss wurde mit warmem Wasser auf 100g aufgefüllt.

### 1.2.2 Herstellung der Gelfomulierungen A2e, A2g, A2o und A2q nicht erfindungsgemäss

Die Aldehyde wurden in 70g 80 bis 90°C heißem Wasser, Isopropanol, Propylenglykol, Diethylenglykol, Propylencarbonat und/oder Glycerin unter Rühren gelöst, dann wurden die für die Einzelformulierungen notwendigen Mengenanteile der Komponenten Texapon^{®} NSO UP, Dehyton^{®} K und/oder Plantacare^{®} 1200 UP hinzu gegeben. Während des Abkühlens wurden die zuvor vorgequollenen Mittel Veegum^{®} Ultra und/oder Carbopol^{®} 2984 unter weiterem Rühren hinzu gegeben. Der Quellvorgang wurde abgewartet. Anschließend wurden die Mittel Natriumsalicylat, Phenoxyethanol und/oder Natriumbenzoat hinzugefügt. Zum Schluss wurde durch Zugabe von Schwefelsäure bzw. Monoethanolamin der für die jeweilige Formulierung angegebene pH-Wert eingestellt und mit kaltem Wasser auf 100g aufgefüllt.

### 1.3 Zusammensetzung der Mittel A3

| Mittel | Komponente |
|---|---|
| A3a | 20%ige Monoethanolaminlösung |
| A3b | konzentrierte Ammoniaklösung in Wasser |
| A3c | 10%ige Natriumhydroxidlösung |
| A3d | 10%ige Argininlösung |
| A3e | 10%ige Kaliumhydroxidlösung |

### 1.4 Zusammensetzung der Mittel A4

| Komponente | A4a | A4b |
|---|---|---|
| Hydrenol^{®} D | 3,5 | - |
| Lorol^{®} | 0,5 | - |
| Eumulgin^{®} B2 | 1,5 | - |
| Texapon^{®} NSO UP | 1,5 | 1,0 |
| Plantacare^{®} 1200 UP | 1,0 | 0,50 |
| Dehyton^{®} K | 0,5 | 1,0 |
| Cremophor^{®} RH 40 | 1,0 | - |
| Tween^{®} 40 | 1,5 | - |
| Propylenglycol | 1,5 | - |
| Diethylenglycol | - | 2,0 |
| Veegum^{®} Ultra | - | 1,35 |
| Carbopol^{®} 2984 | 1,20 | 0,47 |
| Natriumsalicylat | 0,5 | - |
| Phenoxyethanol | 1,0 | - |
| Natriumbenzoat | - | 0,68 |
| Monoethanolamin | Ad pH 9,5 | Ad pH 9,0 |
| Wasser | Ad 100 | Ad 100 |

### 1.4.1 Herstellung der alkalischen Cremeformulierung A4a

Lorol^{®}, Eumulgin^{®} B2 und Hydrenol^{®} D wurden zusammen bei 80°C aufgeschmolzen. In diese Schmelze wurden die für die Einzelformulierungen notwendigen Mengenanteile der Komponenten Texapon^{®} NSO UP, Dehyton^{®} K und Plantacare^{®} 1200 UP eingearbeitet. Nach dem Abkühlen wurden die übrigen Rezepturbestandteile Cremophor^{®} RH 40, Tween^{®} 40 und Propylenglykol hinzugefügt. Anschließend wurde Carbopol^{®} 2984 unter weiterem Rühren langsam hinzu gegeben. Der Quellvorgang wurde abgewartet. Zum Schluss wurden die Mittel Natriumsalicylat und Phenoxyethanol hinzu gegeben und verrührt. Durch die Zugabe von Monoethanolamin wurde der für die Formulierung angegebene pH-Wert eingestellt. Anschließend wurde mit warmem Wasser auf 100g aufgefüllt.

### 1.4.2 Herstellung der alkalischen Gelformulierung A4b

Texapon^{®} NSO UP, Dehyton^{®} K und Plantacare^{®} 1200 UP wurden in 70g 80 bis 90°C heißem Wasser unter Rühren gelöst. Während des Abkühlens wurden die zuvor vorgequollenen Mittel Veegum^{®} Ultra und Carbopol^{®} 2984 unter weiterem Rühren hinzu gegeben. Der Quellvorgang wurde abgewartet. Anschließend wurde Natriumbenzoat hinzugefügt und verrührt. Zum Schluss wurde durch Zugabe von Monoethanolamin der pH-Wert eingestellt und mit warmem Wasser auf 100g aufgefüllt.

### 1.5 Zusammensetzung der Mittel A5

| Komponente | A5a | A5b | A5c | A5d |
|---|---|---|---|---|
| Propylencarbonat | 5,0 | - | - | - |
| Glycerincarbonat | - | 5,0 | - | - |
| Isopropanol | - | - | 5,0 | - |
| Propylenglycol | - | - | - | 5,0 |

### 1.6 Verzeichnis der eingesetzten Handelsprodukte

Die im Rahmen der Beispiele eingesetzten Handelsprodukte sind wie folgt definiert:
- Aerosil^{®} 200: Kieselsäure (INCI-Bezeichnung: Silica) (Degussa)
- Carbopol^{®} 2984: verzweigte Polyacrylsäure (INCI-Bezeichnung: Carbomer) (Noveon)
- Cremophor^{®} RH40: hydriertes Rizinusöl mit ca. 40-45 EO-Einheiten (INCI-Bezeichnung: PEG-40 Hydrogenated Castor Oil) (BASF)
- Dehyton^{®} K: N,N-Dimethyl-N-(C8-18-kokosamidopropyl)ammoniumaceto-betain (ca. 30% Aktivsubstanz; INCI-Bezeichnung: Aqua (Water), Cocamidopropyl Betaine) (Cognis)
- Eumulgin^{®} B2: Cetylstearylalkohol mit ca. 20 EO-Einheiten (INCI-Bezeichnung: Ceteareth-20) (Cognis)
- Hydrenol^{®} D: C₁₆₋₁₈-Fettalkohol (INCI-Bezeichnung: Cetearyl alcohol) (Cognis)
- Lorol^{®}, tech.: C₁₂₋₁₈-Fettalkohol (INCI-Bezeichnung: Coconut alcohol) (Cognis)
- Pemulen^{®} TR-1: verzweigtes Acrylsäurepolymer (INCI-Bezeichnung: Acrylates/C10-30 Alkyl Acrylate Crosspolymer) (Lubrizol)
- Plantacare^{®} 1200 UP: C₁₂₋₁₆-Fettalkohol-1.4-glucosid (ca. 50-53% Aktivsubstanzgehalt; INCI-Bezeichnung: Lauryl Glucoside, Aqua (Water)) (Cognis)
- Solubilisant^{®} Gamma 2428: Tensidmischung (INCI-Bezeichnung: PEG-40 Hydrogenated Castor Oil, Polysorbate 20, Octoxynol-11) (Gattefosse)
- Solubilisant^{®} LRI: ca. 9,5 bis 10,5Gew.-% Wassergehalt; INCI-Bezeichnung: PPG-26-Buteth-26, PEG-40 Hydrogenated Castor Oil (Sensient Cosmetic Technologies)
- Texapon^{®} NSO UP: Laurylethersulfat, Natriumsalz (ca. 27,5% Aktivsubstanz; INCI-Bezeichnung: Sodium Laureth Sulfate) (Cognis)
- Tween^{®} 40: Polysorbate-40 (Uniqema)
- Veegum^{®} Ultra: Magnesium Aluminum Silicate (ca. 8% Wassergehalt; INCI-Bezeichnung: Magnesium Aluminum Silicate) (Vanderbilt)

### 2 Ausfärbungen

### 2.1 Kombinationen zur Erstellung von Nuancen

Es wurden die folgenden Kombinationen ausgefäbt:

| Nuance | Rezepturkombination | AusfärbepH-Wert | Farbe |
|---|---|---|---|
| 1 | A1a/A2a/A4a | 8,5 | Orangerot |
| 2 | A1b/A2a/A4a | 7,5 | Kupfer |
| 3 | A1c/A2a/A3c | 8,4 | Kupferorange |
| 4 | A1a/A2b/A4a | 7,8 | Orange |
| 5 | A1b/A2b/A4a | 7,9 | Orangegelb |
| 6 | A1a/A2c/A4b/A5a | 8,0 | Schwarzbraun |
| 7 | A1b/A2c/A4b/A5c | 8,2 | Dunkelbraun |
| 8 | A1c/A2c/A3c/A5b | 8,1 | Mittelbraun |
| 9 | A1a/A2d/A3d | 9,5 | Schwarzbraun |
| 10 | A1b/A2d/A3e | 8,3 | Mittelbraun |
| 11 | A1c/A2d/A3d/A5d | 8,7 | Hellbraun |
| 12 | A1a/A2e/A3b | 7,7 | Rotkupfer |
| 13 | A1b/A2e/A3c | 8,6 | Orangekupfer |
| 14 | A1a/A2f/A3a | 7,6 | Schwarzbraun |
| 15 | A1b/A2f/A3e | 8,0 | Dunkelbraun |
| 16 | A1d/A2f/A3d | 8,8 | Schwarzbraun |
| 17 | A1e/A2f/A3a | 8,5 | Dunkelbraun |
| 18 | A1d/A2g/A3b | 7,5 | Schwarz |
| 19 | A1e/A2g/A3c | 8,4 | Schwarz |
| 20 | A1a/A2h/A4a/A5b | 7,8 | Dunkelbraun |
| 21 | A1f / A2h / A3e / A5a | 7,9 | Dunkelbraun |
| 22 | A1g/A2h/A3a/A5c | 8,0 | Dunkelbraun |
| 23 | A1a/A2i/A4b | 8,2 | Schwarz |
| 24 | A1b/A2i/A4b | 8,1 | Schwarz |
| 25 | A1f/A2i/A3d | 9,5 | Schwarz |
| 26 | A1g/A2i/A3e | 8,3 | Schwarz |
| 27 | A1a/A2j/A4b | 8,7 | Schwarz |
| 28 | A1b/A2j/A4b | 7,7 | Schwarz |
| 29 | A1f/A2j/A3c | 8,6 | Schwarz |
| 30 | A1g/A2j/A3a | 7,6 | Schwarz |
| 31 * | A1a/A2k/A4a | 8,5 | Orangerot |
| 32 * | A1b/A2k/A4a | 7,5 | Kupfer |
| 33 * | A1c/A2k/A3c | 8,4 | Kupferorange |
| 34 | A1a/A2l/A4a | 7,8 | Orange |
| 35 | A1b/A2l/A4a | 7,9 | Orangegelb |
| 36 * | A1a/A2m/A4b/A5a | 8,0 | Schwarzbraun |
| 37 * | A1b/A2m/A4b/A5c | 8,2 | Dunkelbraun |
| 38 * | A1c/A2m/A3c/A5b | 8,1 | Mittelbraun |
| 39 * | A1a/A2n/A3d | 9,5 | Schwarzbraun |
| 40 * | A1b/A2n/A3e | 8,3 | Mittelbraun |
| 41 * | A1c/A2n/A3d/A5d | 8,7 | Hellbraun |
| 42 * | A1a/A2o/A3b | 7,7 | Rotkupfer |
| 43 * | A1b/A2o/A3c | 8,6 | Orangekupfer |
| 44 * | A1a/A2p/A3a | 7,6 | Schwarzbraun |
| 45 * | A1b/A2p/A3e | 8,0 | Dunkelbraun |
| 46 * | A1d/A2p/A3d | 8,8 | Schwarzbraun |
| 47 * | A1e/A2p/A3a | 8,5 | Dunkelbraun |
| 48 * | A1d/A2q/A3b | 7,5 | Schwarz |
| 49 * | A1e/A2q/A3c | 8,4 | Schwarz |
| 50 * | A1a / A2r / A4a / A5b | 7,8 | Dunkelbraun |
| 51 * | A1f/A2r/A3e/A5a | 7,9 | Dunkelbraun |
| 52 * | A1g / A2r / A3a / A5c | 8,0 | Dunkelbraun |
| 53 * | A1a/A2s/A4b | 8,2 | Schwarz |
| 54 * | A1b/A2s/A4b | 8,1 | Schwarz |
| 55 * | A1f / A2s/ A3d | 9,5 | Schwarz |
| 56 * | A1g/A2t/A3e | 8,3 | Schwarz |
| 57 * | A1a/A2t/A4b | 8,7 | Schwarz |
| 58 * | A1b/A2t/A4b | 7,7 | Schwarz |
| 59 * | A1f/A2t/A3c | 8,6 | Schwarz |
| 60 * | A1g/A2t/A3a | 7,6 | Schwarz |

| | | | |
|---|---|---|---|
| * nicht erfindungsgemäss | | | |

### 2.1.1 Ausfärbungsdetails

Zur Herstellung der gebrauchsfertigen Färbemischung der Nuancen 16 bis 19 sowie 46 bis 49 wurden die Mittel (A1) und (A2) im Gewichtsverhältnis 1:1 innig vermischt. Direkt anschließend wurde soviel von der Zubereitung (A3) hinzugefügt, bis der in Tabelle angegebene pH-Wert der Mischung erreicht wurde.

Zur Herstellung der gebrauchsfertigen Färbemischung der Nuancen 11 und 41 wurden die Mittel (A1) und (A2) im Gewichtsverhältnis 1:1 miteinander vermischt und zusätzlich das Mittel (A5) hinzugefügt. Anschließend wurde die Mischung innig vermischt und soviel von der Zubereitung (A3) hinzugefügt, bis der in Tabelle angegebene pH-Wert der Mischung erreicht wurde.

Zur Herstellung der gebrauchsfertigen Färbemischung, der Nuancen 9, 10, 12 bis 15, 39, 40 sowie 42 bis 45 wurde das Mittel (A1) in das Mittel (A2) gegeben innig vermischt. Direkt anschließend wurde soviel von der Zubereitung (A3) hinzugefügt, bis der in Tabelle angegebene pH-Wert der Mischung erreicht wurde.

Zur Herstellung der gebrauchsfertigen Färbemischung, der Nuancen 3, 25, 26, 29, 30, 33, 55, 56 59 und 60 wurde das Mittel (A2) zum Mittel (A1) gegeben und anschließend innig vermischt. Direkt anschließend wurde soviel von der Zubereitung (A3) hinzugefügt, bis der in Tabelle angegebene pH-Wert der Mischung erreicht wurde.

Zur Herstellung der gebrauchsfertigen Färbemischung, der Nuancen 8, 21, 22, 38, 51 und 52 wurden das Mittel (A2) zum Mittel (A1) gegeben. Das Mittel (A5) wurde zusätzlich hinzugefügt und alles innig vermischt. Direkt anschließend wurde soviel von der Zubereitung (A3) hinzugefügt, bis der in Tabelle angegebene pH-Wert der Mischung erreicht wurde.

Zur Herstellung der gebrauchsfertigen Färbemischung der Nuancen 1, 2, 4, 5, 23, 24, 27, 28, 31, 32, 34, 35, 54, 57 und 58. wurden die Mittel (A1) und (A2) zum Mittel (A4) gegeben und innig vermischt. Es stellte sich der in Tabelle angegebene pH-Wert ein.

Zur Herstellung der gebrauchsfertigen Färbemischung der Nuancen 6, 7, 20, 36, 37 und 50 wurde die Mittel (A1) und (A2) zum Mittel (A4) gegeben. Zusätzlich wurde das Mittel (A5) hinzu gegeben. Nach inniger Vermischung stellte sich der in Tabelle angegebene pH-Wert ein.

Alle erhaltenen gebrauchsfertigen Färbemittel wurden im Verhältnis 3 g Farbmischung zu 1 g Haar (Kerling naturweiß) aufgebracht. Nach einer Einwirkzeit von 30 Minuten bei 32°C wurden die Strähnen mit lauwarmem Wasser ausgespült und danach im warmen Luftstrom (30 bis 40°C) getrocknet. Die Färbungen wurden visuell unter einer Tageslichtlampe beurteilt.

## Patentansprüche

1. Mittel zur Färbung keratinischer Fasern, enthaltend in einem kosmetisch akzeptablen Träger mindestens eine C,H-acide Verbindung, ausgewählt aus
- den physiologisch verträglichen Salzen des 1-Allyl-1,2-dihydro-3,4,6-trimethyl-2-oxo-pyrimidiniums, insbesondere 1-Allyl-1,2-dihydro-3,4,6-trimethyl-2-oxo-pyrimidinium-bromid und
- den physiologisch verträglichen Salzen des 1,2-Dihydro-1,3,4,6-tetramethyl-2-oxo-pyrimidiniums, insbesondere 1,2-Dihydro-1,3,4,6-tetramethyl-2-oxo-pyrimidinium-hydrogensulfat,
sowie als reaktive Carbonylverbindung, mindestens
- 2,3-Dihydro-1,5-dimethyl-3-oxo-2-phenyl-1H-pyrazol-4-carboxaldehyd

2. Mittel nach Anspruch 1, **dadurch gekennzeichnet, dass** es weiterhin 4-Hydroxy-2-methoxybenzaldehyd enthält.

3. Mittel nach einem der Ansprüche 1 oder 2, **dadurch gekennzeichnet, dass** es 2-(Cyanmethyl)-benzimidazol als weitere C,H-acide Verbindung enthält.

4. Mittel nach einem der Ansprüche 1 bis 3, **dadurch gekennzeichnet, dass** es als weitere reaktive Carbonylverbindung 3,4-Dihydroxy-5-methoxybenzaldehyd, 2-Chlor-3,4-dihydroxybenzaldehyd und/oder 2-Brom-3,4-dihydroxybenzaldehyd enthält.

5. Mittel nach einem der Ansprüche 1 bis 4, **dadurch gekennzeichnet, dass** die reaktiven Carbonylverbindungen jeweils in einer Menge von 0,03 bis 65 mmol, insbesondere von 1 bis 40 mmol, bezogen auf 100 g des gesamten Färbemittels, enthalten sind.

6. Mittel nach einem der Ansprüche 1 bis 5, **dadurch gekennzeichnet, dass** die C,H-aciden Verbindungen vorzugsweise in einer Menge von 0,03 bis 65 mmol, insbesondere von 1 bis 30 mmol, bezogen auf 100 g des gesamten Färbemittels, enthalten sind.

7. Verfahren zum Färben von keratinhaltigen Fasern, insbesondere menschlichen Haaren, worin ein Färbemittel, gemäß einem der Ansprüche 1 bis 6, sowie übliche kosmetische Inhaltsstoffe, auf die keratinhaltigen Fasern aufgebracht, einige Zeit, üblicherweise ca. 15-30 Minuten, auf der Faser belassen und anschließend wieder ausgespült oder mit einem Shampoo ausgewaschen wird.

## Claims

1. Agent for colouring keratinic fibres, comprising in a cosmetically acceptable carrier at least one C,H-acidic compound, selected from
- the physiologically acceptable salts of 1-allyl-1,2-dihydro-3,4,6-trimethyl-2-oxopyrimidinium, in particular 1-allyl-1,2-dihydro-3,4,6-trimethyl-2-oxo-pyrimidinium bromide
and
- the physiologically acceptable salts of 1,2-dihydro-1,3,4,6-tetramethyl-2-oxopyrimidinium, in particular 1,2-dihydro-1,3,4,6-tetramethyl-2-oxo-pyrimidinium hydrogen sulfate,
as well as a reactive carbonyl compound, at least
- 2,3-dihydro-1,5-dimethyl-3-oxo-2-phenyl-1*H*-pyrazole-4-carboxaldehyde.

2. Agent according to claim 1, **characterised in that** it further comprises 4-hydroxy-2-methoxybenzaldehyde.

3. Agent according to one of claims 1 or 2, **characterised in that** it comprises 2-(cyanomethyl)benzimidazole as the further C,H-acidic compound.

4. Agent according to one of claims 1 to 3, **characterised in that** it comprises 3,4-dihydroxy-5-methoxybenzaldehyde, 2-chloro-3,4-dihydroxybenzaldehyde and/or 2-bromo-3,4-dihydroxybenzaldehyde as the further reactive carbonyl compound.

5. Agent according to one of the claims 1 to 4, **characterised in that** the reactive carbonyl compounds are each comprised in an amount of 0.03 to 65 mmol, in particular from 1 to 40 mmol, based on 100 g of the total coloring agent.

6. Agent according to one of the claims 1 to 5, **characterised in that** the C,H-acidic compounds are comprised in an amount of 0.03 to 65 mmol, in particular from 1 to 30 mmol, based on 100 g of the total coloring agent.

7. Method for dyeing keratin-containing fibres, especially human hair, in which a coloring agent according to one of claims 1 to 6, together with conventional cosmetic ingredients, is applied onto the keratin-containing fibres, left on the fibres for some time, usually about 15-30 minutes, and then rinsed out again or washed out with a shampoo.

## Revendications

1. Agent pour la teinture de fibres kératiniques, contenant, dans un support acceptable du point de vue cosmétique, au moins un composé à C,H acide choisi parmi
- les sels physiologiquement acceptables du 1-allyl-1,2-dihydro-3,4,6-triméthyl-2-oxo-pyrimidinium, en particulier le bromure du 1-allyl-1,2-dihydro-3,4,6-triméthyl-2-oxo-pyrimidinium ;
et
- les sels physiologiquement acceptables du 1,2-dihydro-1,3,4,6-tétraméthyl-2-oxo-pyrimidinium, en particulier l'hydrogénosulfate du 1,2-dihydro-1,3,4,6-tétraméthyl-2-oxo-pyrimidinium ;
et à titre de composé carbonyle réactif, au moins
- du 2,3-dihydro-1,5-diméthyl-3-oxo-2-phényl-1H-pyrazol-4-carboxaldéhyde.

2. Agent selon la revendication 1, **caractérisé en ce qu'**il contient en outre le 4-hydroxy-2-méthoxybenzaldéhyde.

3. Agent selon l'une quelconque des revendications 1 ou 2, **caractérisé en ce qu'**il contient du 2-(cyanométhyl)-benzimidazole à titre de composé supplémentaire à C,H acide.

4. Agent selon l'une quelconque des revendications 1 à 3, **caractérisé en ce qu'**il contient, à titre de composé carbonyle réactif supplémentaire, le 3,4-dihydroxy-5-méthoxybenzaldéhyde, le 2-chloro-3,4-dihydroxy-benzaldéhyde et/ou le 2-bromo-3,4-dihydroxybenzaldéhyde.

5. Agent selon l'une quelconque des revendications 1 à 4, **caractérisé en ce qu'**il contient les composés carbonyle réactifs à chaque fois en une quantité de 0,03 à 65 millimoles, en particulier de 1 à 40 millimoles, rapportés à 100 g de l'agent de teinture total.

6. Agent selon l'une quelconque des revendications 1 à 5, **caractérisé en ce qu'**il contient les composés à C,H acide de préférence en une quantité de 0,03 à 65 millimoles, en particulier de 1 à 30 millimoles, rapportés à 100 g de l'agent de teinture total.

7. Procédé pour la teinture de fibres kératiniques, en particulier de cheveux humains, dans lequel on applique sur les fibres kératiniques un agent de teinture, selon l'une quelconque des revendications 1 à 6, ainsi que des constituants cosmétiques habituels, on l'abandonne sur les fibres kératiniques pendant un certain temps, habituellement d'environ 15 à 30 minutes, et on l'en élimine ensuite par rinçage ou par lavage avec un shampooing.
